(19) **Europäisches Patentamt European Patent Office Office européen des brevets**

(11) **EP 2 120 792 B1**

(12) **FASCICULE DE BREVET EUROPEEN**

(45) Date de publication et mention de la délivrance du brevet:
**10.06.2015 Bulletin 2015/24**

(21) Numéro de dépôt: **08761861.7**

(22) Date de dépôt: **08.02.2008**

(51) Int Cl.:
***A61F 2/16*** *(2006.01)*

(86) Numéro de dépôt international:
**PCT/FR2008/000160**

(87) Numéro de publication internationale:
**WO 2008/119894 (09.10.2008 Gazette 2008/41)**

(54) **IMPLANT OCULAIRE ACCOMMODATIF**

AKKOMMODATIVES AUGENIMPLANTAT

ACCOMMODATIVE OCULAR IMPLANT

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **01.03.2007 FR 0753575**

(43) Date de publication de la demande:
**25.11.2009 Bulletin 2009/48**

(73) Titulaires:
• **Centre National de la Recherche Scientifique (CNRS)**
**75794 Paris Cedex 16 (FR)**
• **Michel, François**
**74200 Thonon Les Bains (FR)**

(72) Inventeurs:
• **MICHEL, François**
**74200 Thonon Les Bains (FR)**
• **BUISINE, Jean-Marc**
**59830 Louvil (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A-85/05466     WO-A-94/23334**
**WO-A-03/007851    FR-A1- 2 777 091**
**US-A- 4 373 218    US-A1- 2006 136 055**

**Description**

L'invention se rapporte à un dispositif pour la correction visuelle au niveau d'un oeil comprenant :

- un convertisseur apte à générer un signal électrique et/ou magnétique et/ou électromagnétique en réponse à l'énergie mécanique générée par un mouvement de l'oeil;
- une lentille souple agencée pour être positionnée au niveau de l'oeil,

le convertisseur étant agencé par rapport à la lentille de sorte que les propriétés optiques de la lentille changent sous l'action du signal électrique et/ou magnétique et/ou électromagnétique lors du mouvement de l'oeil.

Pour un convertisseur apte à générer un signal électrique, un tel dispositif est décrit dans la demande internationale WO 2004/004605 Ce document se rapporte à un dispositif du type implant oculaire dans lequel la lentille souple est une lentille intraoculaire qui peut être implantée dans l'oeil d'un patient, par exemple lorsque ce patient est atteint de cataracte. Dans ce document, un convertisseur permet de transformer l'énergie mécanique générée par le mouvement d'un oeil, en un signal électrique. Ce convertisseur comprend notamment des capteurs de pression qui détectent le mouvement de convergence des yeux. Ce signal électrique est alors fourni à la lentille sous forme de tension électrique. Sous l'effet de cette tension électrique, le rayon de courbure de la lentille subit une modification par effet piézo-électrique. Cette modification est provoquée par un fil en boucle ouverte mobile sous l'effet d'une tension électrique, qui ceinture la lentille et modifie donc son rayon de courbure lorsqu'une tension est appliquée. Un tel dispositif est pseudo-accommodatif au sens où une accommodation est réalisée en réaction au mouvement des deux yeux lorsque les yeux convergent lors du passage d'une vision de loin à une vision de près. L'effet de modification de la courbure est alors réalisé lorsque cette condition de convergence est détectée.

Dans la demande susmentionnée, le mouvement déclenchant une modification des propriétés optiques de la lentille est un mouvement particulier des deux yeux correspondant à une convergence. Toutefois, dans le cadre de l'invention, le mouvement de l'oeil peut être n'importe quel mouvement, par exemple défini par une variation de l'orientation dans l'espace de l'axe optique d'un oeil. Des propriétés particulières des éléments de l'invention pourront alors être définies en fonction d'un mouvement particulier. Un tel dispositif a l'avantage important que c'est l'énergie mécanique liée au mouvement des yeux qui est transformée en énergie électrique susceptible de servir pour modifier les propriétés optiques de la lentille par effet piézo-électrique. L'utilisation de l'énergie mécanique comme source d'énergie initiale, notamment sous forme de pression ou de variation de longueur ou de déformation, a l'avantage de ne pas être lié à l'âge de l'utilisateur. En effet, quel que soit cet âge, le mouvement de l'oeil reste le même pour converger.

Bien qu'avantageux pour générer un implant pseudo-accommodatif pour la correction visuelle, un tel dispositif possède toutefois un certain nombre d'inconvénients.

En particulier, il est difficile de modifier la courbure d'une lentille intra-oculaire car celle-ci se charge de dépôts fibrotiques. La lentille colle donc aux tissus sur lesquels elle est placée, ce qui empêche de modifier la courbure de façon contrôlée et satisfaisante de façon reproductible dans le temps.

Par ailleurs, dans la demande internationale précitée, la structure et la composition de la lentille sont adaptées pour que la courbure soit modifiée en réponse au mouvement des yeux. Cette composition n'est alors plus adaptée pour d'autres types de modification des propriétés optiques de la lentille.

En outre, il est connu que, dans le cadre d'une lentille intra-oculaire la meilleure façon d'insérer la lentille est de la plier, de l'insérer dans une incision de faible taille dans l'oeil, et de laisser la lentille reprendre une forme désirée. Du fait des moyens utilisés pour modifier la courbure dans la demande internationale susmentionnée, une telle procédure est rendue plus complexe.

L'invention vise notamment à pallier ces inconvénients.

Un problème résolu par l'invention est donc de fournir un dispositif pour la correction visuelle tel que décrit précédemment, qui puisse réagir à l'énergie mécanique générée par un mouvement de l'oeil, et pour lequel les modifications des propriétés optiques de la lentille soient simplifiées et mieux contrôlées. Il convient également que les propriétés de la lentille soient stables en l'absence de tout signal électrique et/ou magnétique et/ou électromagnétique.

On connaît également le document WO-A-03/007851 qui enseigne un dispositif tel que mentionné ci-dessus.

Dans ce document, des cristaux liquides sont insérés dans une poche pour former la lentille souple. Sous l'action d'une contraction de l'iris, l'orientation des cristaux liquides peut être modifiée de sorte que les propriétés optiques de la lentille changent.

Toutefois, la nature de la poche ou de la lentille n'est pas mentionnée dans ce document.

Or, comme mentionné ci-dessus, dans le cadre d'une lentille intraoculaire la meilleure façon d'insérer la lentille est de la plier, de l'insérer dans une incision de faible taille dans l'oeil, et de laisser la lentille reprendre une forme désirée. Dès lors, en utilisant une poche quelconque comprenant des cristaux liquides, ce pliage provoquerait une modification de l'arrangement moléculaire des cristaux liquides. Cette modification peut alors être irréversible, rendant ainsi le dispositif inutilisable.

Au vu du document susmentionné, l'invention vis donc à améliorer la lentille souple.

Ces problèmes sont résolus par un dispositif pour la correction visuelle au niveau d'un oeil tel que précédemment décrit, et caractérisé en ce que la lentille souple comprend un matériau polymère dans lequel est inclus un matériau ayant un indice de réfraction susceptible de varier sous l'action du signal électrique et/ou magnétique et/ou électromagnétique en réponse au mouvement de l'oeil.

Grâce à un tel dispositif, lors des mouvements de l'oeil, l'indice de réfraction du matériau peut varier simplement sous l'action du signal électrique et/ou magnétique et/ou électromagnétique ce qui modifie les propriétés optiques de la lentille. Le dispositif est donc susceptible de s'adapter en réaction aux mouvements de l'oeil. L'insertion du matériau à indice de réfraction variable dans le polymère permet d'obtenir cette modification de façon adaptée en réponse à un mouvement de l'oeil. Il est en effet possible d'obtenir une certaine stabilité du matériau à indice variable dans le polymère en l'absence de signal électrique et/ou magnétique et/ou électromagnétique, tout en ayant une bonne réactivité du matériau au signal appliqué en cas de mouvement de l'oeil.

Selon l'invention, la courbure de la lentille n'est pas nécessairement modifiée, ce qui permet d'éviter les problèmes susmentionnés liés à la fibrose des tissus au contact de la lentille. Le fait que le matériau à indice de réfraction variable soit dans la lentille permet alors un contrôle simple des propriétés de modification de la lentille puisque l'environnement extérieur de la lentille, tel que des fibroses, n'influe pas sur les modifications des propriétés optiques.

Dans le domaine des lentilles intra-oculaires à puissance optique variable, on connaît également le brevet américain US 4,373,218. Ce brevet enseigne un dispositif pour la correction visuelle au niveau d'un oeil comprenant une lentille, la lentille comprenant des cristaux liquides. Ces cristaux liquides ont la propriété connue de s'orienter sous l'effet d'un champ électrique ou magnétique ou électromagnétique. Dans le brevet susmentionné, selon un mode de réalisation, des électrodes sont placées dans l'oeil de l'utilisateur, permettent de détecter une accommodation. Toutefois, dans ce brevet, ce n'est pas l'énergie mécanique liée au mouvement des yeux qui est détecté, mais un potentiel électrique généré par les muscles lors de l'accommodation. L'utilisation de l'énergie générée par les muscles lors de l'accommodation n'est pas satisfaisante car celle-ci n'est pas stable, et varie notamment avec l'âge de l'utilisateur, ce qui nécessitera donc de modifier le dispositif régulièrement. De plus, dans le brevet susmentionné le mode d'utilisation utilise seulement l'effet d'un champ électrique. Toutefois, dans le cadre de l'invention, les effets utilisés peuvent être électriques et/ou magnétiques et/ou électromagnétiques

En outre, les cristaux liquides décrits dans le brevet susmentionné ne sont pas adaptés pour réagir de façon satisfaisante en réponse à un mouvement de l'oeil. En effet, ils ne sont pas plongés dans un milieu permettant à la fois une bonne stabilité orientationnelle et bonne homogénéité spatiale et temporelle en l'absence de champ électromagnétique et une bonne réaction spatiale et bonne homogénéité en présence de champ électrique. Au contraire, dans l'invention, le fait de positionner le matériau à indice variable dans un matériau polymère permet d'obtenir cet avantage. De plus, l'invention permet d'obtenir cet avantage en présence de champs électriques et/ou magnétiques et/ou électromagnétiques

Dans ce brevet, ce n'est donc pas l'énergie mécanique liée au déplacement d'un oeil qui génère le signal électrique nécessaire pour modifier les propriétés optiques des cristaux liquides. Ce brevet n'utilise donc aucun convertisseur. Du fait de l'utilisation des seuls potentiels électriques générés par les zones musculaires des yeux, le dispositif décrit dans ce brevet n'est donc pas satisfaisant dans la pratique.

Le brevet susmentionné ne résout donc nullement le problème susmentionné lié à une modification nécessaire en réaction à un mouvement de l'oeil.

Par ailleurs, dans le brevet susmentionné, il est complexe de donner une forme désirée à la lentille. Au contraire, l'utilisation d'un matériau polymère pouvant servir de matrice permet d'imposer une telle forme de façon simple, notamment par élaboration de plaques de composites à découper et à usiner. En outre, selon l'invention, il est possible de fournir une orientation prédéfinie à des cristaux liquides dès l'élaboration de la lentille, et non pas seulement lorsque la lentille est positionnée dans l'oeil. Enfin, la lentille selon l'invention permet, grâce à l'utilisation d'un matrice de polymère comprenant le matériau à indice variable, par exemple sous la forme de cristaux liquides, de maintenir une orientation satisfaisante des cristaux liquides même si la lentille doit être pliée ou manipulée avant son introduction dans l'oeil d'un utilisateur.

On connaît également la demande de brevet EP-A-1 068 555, dans laquelle on décrit un dispositif pour la correction visuelle au niveau d'un oeil comprenant une lentille, la lentille comprenant des cristaux liquides. Les cristaux liquides ont la propriété connue de s'orienter sous l'effet d'un champ de contraintes. Dans cette demande, le matériau utilisé pour fabriquer la lentille comprend un polymère cristaux liquides tridimensionnel. Or, dans un tel polymère cristaux liquides, les parties liquides cristallines des molécules sont fortement figées, de sorte que l'effet d'orientation des parties liquides cristallines n'est obtenu que sous une contrainte forte de l'ensemble du polymère. C'est pourquoi, dans cette demande, la modification de l'orientation est réalisée sous l'action d'une contrainte mécanique, par exemple lors d'un clignement de paupière. Les polymères cristaux liquides décrits dans cette demande ne sont donc pas susceptibles de s'orienter sous l'action d'un signal électrique et ou/ magnétique et/ou électromagnétiques, et en particulier, ils ne sont pas susceptibles de s'orienter sous l'action du signal électrique et/ ou magnétiques et/ou électromagnétiques généré par le mouvement de l'oeil.

On décrit maintenant d'autres caractéristiques avantageuses de l'invention.

Lorsqu'il est électrique, le signal généré par le convertisseur suite au mouvement de l'oeil peut correspondre à une tension relativement faible, typiquement de l'ordre de 1 volt à 5 volts. Il est donc avantageux que, l'effet de réaction au mouvement de l'oeil se produise même lorsque la tension générée par le mouvement de l'oeil est faible. Seules des faibles tensions sont en effet compatibles avec le corps humain.

Cet avantage supplémentaire est atteint selon un mode de réalisation de l'invention par la caractéristique selon laquelle le matériau comprend des cristaux liquides ayant une orientation susceptible de varier sous l'action d'un signal électrique et/ou magnétique et/ou électromagnétique lors du mouvement de l'oeil.

Selon ce mode réalisation, suite au mouvement de l'oeil, le signal mécanique peut générer un signal électrique et ou magnétique et/ou électromagnétique et les cristaux liquides s'orientent sous l'action des champs électriques et ou magnétiques et/ou électromagnétiques associés, ce qui a pour effet de modifier l'indice de réfraction de la lentille. La lentille se comporte donc comme un matériau biréfringent qui réagit aux mouvements de l'oeil.

Par ailleurs, les cristaux liquides ont la propriété de s'orienter facilement sous l'action d'une tension électrique, même faible, typiquement de l'ordre du volt. Pour des applications à un implant intra-oculaire, un tel dispositif permet donc d'utiliser les faibles tensions générées par le mouvement de l'oeil.

De façon avantageuse, la lentille comprend un composite du matériau polymère et des cristaux liquides. Grâce à ce composite, la lentille peut être manipulée facilement sans modifier de façon irréversible l'arrangement moléculaire des cristaux liquides, ce qui rendrait le dispositif inutilisable.

Selon un mode de réalisation, le composite est une matrice polymère gonflée par des cristaux liquides. Un tel mode de réalisation a l'avantage d'être facilement réalisable. On peut également ajouter une enveloppe de protection en polymère ayant pour fonction de protéger les cristaux liquides de la matrice de polymère de sorte à éviter qu'ils fuient de la matrice de polymère.

Selon un autre mode de réalisation, le composite est un gel de polymère et de cristaux liquides. Ce mode de réalisation a l'avantage de permettre un changement d'orientation aisé des cristaux liquides pour des tensions seuils relativement faibles compatibles avec l'énergie générée par le mouvement de l'oeil. Dans un gel de polymère, le mouvement des cristaux liquides est en effet facilité. Comme précédemment, on peut également ajouter une enveloppe de protection en polymère.

Selon encore un autre mode de réalisation de l'invention, le composite est une matrice de polymère comprenant une dispersion de gouttelettes de cristaux liquides. Les gouttelettes peuvent être des micro-gouttelettes ou des nano-gouttelettes. Ce mode de réalisation a l'avantage de pouvoir présenter une diffusion optique de la lentille compatible avec les applications envisagées. Ce mode de réalisation a également l'avantage que la tension électrique à appliquer pour provoquer le changement d'orientation des cristaux liquides est relativement faible et compatible avec l'énergie générée par le mouvement de l'oeil.

Dans ces trois modes de réalisation, le fait que les cristaux liquides soient associés à un polymère sous la forme d'un composite a l'avantage de permettre une souplesse de la lentille, permettant notamment son pliage pour l'introduction dans l'oeil dans le cas des lentilles intra-oculaires, de sorte que l'incision nécessaire dans l'oeil d'un patient est relativement faible. Les propriétés de mémoire de forme du polymère permettent alors à la lentille de reprendre sa forme utile correspondant sensiblement à un cristallin suite au pliage et à l'introduction de la lentille.

Par ailleurs, selon l'invention, les cristaux liquidés peuvent être soit des cristaux liquides nématiques, ayant l'avantage d'être les cristaux les plus connus et donc permettant une réalisation simple, soit des cristaux liquides ferroélectriques, ayant l'avantage d'avoir une bonne réponse diélectrique, et donc une meilleure réaction à la tension appliquée, même si celle-ci est relativement faible lors du mouvement de l'oeil.

Le polymère associé aux cristaux liquides dans la lentille peuvent être un polyacrylate ayant un indice optique $n_{poly}$ sensiblement égal à l'indice optique moyen des cristaux liquides $n_{CL}$ de sorte à éviter les phénomènes de diffusion au sein de la lentille.

On décrit maintenant d'autres caractéristiques avantageuses des électrodes transparentes selon le mode de réalisation particulier de l'invention précédemment décrit.

Selon un mode de réalisation de ces électrodes, elles comprennent un matériau d'oxyde mixte indium/ étain, connu sous l'acronyme anglais ITO. Ce matériau a l'avantage d'être un matériau conducteur et transparent usuel.

Selon un mode de réalisation de ces électrodes, elles comprennent un polymère conducteur. L'avantage de ces polymères conducteurs est qu'ils ont une bonne compatibilité biologique et peuvent donc être facilement utilisés dans le cas notamment d'un dispositif intraoculaire. Ce polymère conducteur a également l'avantage de pouvoir être utilisé comme enveloppe de protection de la lentille associée aux cristaux liquides pour retenir les cristaux dans le polymère.

En outre, dans les modes de réalisation dans lesquels une tension est appliquée aux cristaux liquides de sorte à modifier leur orientation, il est avantageux que les cristaux liquides changent d'orientation de façon commune, sans par exemple qu'une partie des cristaux tourne dans un sens et qu'une autre partie tournent dans un autre sens.

Cet avantage supplémentaire est obtenu par la caractéristique selon laquelle, dans la lentille, indépendamment de l'application de toute tension, les cristaux liquides sont inclinés par rapport à une orientation homéotrope. Cette inclinaison correspondant à une pré-orientation des cristaux liquides peut être obtenue par l'application d'un champ électrique de pré-orientation lors de la fabrication de la lentille, et notamment lors de la polymérisation de la lentille.

On peut aussi obtenir cette orientation préférentielle par des traitements chimiques particuliers des surfaces externes de la lentille par exemple par dépôt de molécules induisant une orientation spécifique des molécules de cristaux liquides par liaisons chimiques faibles.

En outre, l'indice de réfraction du polymère est sensiblement égal à l'indice de réfraction des cristaux liquides de sorte à obtenir un milieu transparent.

Par ailleurs, il est également avantageux que le convertisseur puisse générer simplement une tension électrique et ne gêne pas la vision d'un utilisateur du dispositif selon l'invention lorsque ce dernier est placé au niveau de l'oeil d'un utilisateur.

Cet avantage supplémentaire est atteint selon un mode de réalisation de l'invention par la caractéristique selon laquelle le convertisseur comprend une paire d'électrodes transparentes dans le domaine du visible, les électrodes de la paire d'électrodes étant positionnées de part et d'autre de la lentille.

De la sorte, les électrodes forment un condensateur autour de la lentille. Ce condensateur permet d'appliquer la tension au matériau à indice de réfraction variable, de sorte à faire varier cet indice de réfraction lors d'un mouvement de l'oeil. Le fait que les électrodes soient transparentes dans le visible permet en outre de ne pas gêner la vision d'un utilisateur du dispositif.

En outre, afin de fournir un dispositif dont la réponse au mouvement de l'oeil soit satisfaisante de sorte à modifier de façon correcte les propriétés optiques de la lentille, le convertisseur peut comprendre un capteur de pression et un transducteur apte à transformer une pression en un signal électrique et/ou magnétique et/ou électromagnétique. De la sorte, lorsque l'oeil bouge, la pression générée par ce mouvement est détectée par le capteur de pression, et le transducteur va ensuite générer le signal électrique et/ou magnétique et/ou électromagnétique. De façon plus générale, le convertisseur peut comprendre un moyen pour détecter l'énergie mécanique provoquée par une rotation d'un oeil ou des yeux.

Le convertisseur comprend également de préférence un calculateur, de sorte à adapter le signal électrique et/ou magnétique et/ou électromagnétique généré en fonction de l'énergie mécanique détectée, notamment la pression. Les capteurs de pression et le calculateur communiquent de préférence par ultrason.

En outre, dans le cas d'un mouvement d'accommodation des yeux d'un utilisateur correspondant à une convergence des yeux, il est avantageux que les propriétés optiques de la lentille ne soient pas modifiées en dehors du parcours accommodatif, mais commence à partir d'un point appelé punctum remotum correspondant au point à partir duquel un oeil sain commence l'accommodation. De la sorte, l'effet de simulation de l'accommodation se rapproche fortement d'une accommodation réelle. Ce punctum remotum est situé environ à cinq mètres des yeux.

Cet avantage supplémentaire est obtenu selon un mode de réalisation de l'invention par la caractéristique selon laquelle le convertisseur est agencé de sorte que la tension électrique seuil de modification d'orientation des cristaux liquides soit atteinte lorsqu'un mouvement d'accommodation des yeux démarre au punctum remotum.

En effet, il est connu que pour des cristaux liquides, l'effet de modification de l'orientation ne commence qu'à partir d'une tension électrique seuil de modification d'orientation. A partir de cette tension seuil, les cristaux commencent alors à orienter leur moment dipolaire. Pour des cristaux liquides dans un film mince, au-delà de cette tension seuil, la variation de l'orientation des cristaux liquides est alors sensiblement proportionnelle à la tension électrique appliquée. Cet effet est connu sous le nom d'effet Fréedericksz.

En utilisant cet effet de tension électrique seuil, on agence alors le convertisseur pour que l'effet d'orientation ne commence que lorsque le mouvement des yeux a atteint le punctum remotum. Ceci permet donc d'obtenir une bonne simulation de l'accommodation lors d'un mouvement d'accommodation des yeux correspondant à une convergence. Cet avantage est notamment atteint du fait que le signal électrique et/ou magnétique et/ou électromagnétique généré est issu de l'énergie mécanique due à la convergence des yeux, ce qui permet d'adapter ce signal en fonction de ce mouvement.

En moyenne, le punctum remotum est situé à environ cinq mètres des yeux. Ainsi, le convertisseur peut être agencé de sorte que la tension seuil de modification d'orientation des cristaux liquides soit atteinte lorsque l'intersection des axes optiques des deux yeux se coupe à une distance d'environ cinq mètres par rapport aux yeux.

Cet agencement particulier du convertisseur peut être obtenu en programmant le calculateur précédemment mentionné de sorte à atteindre ce résultat. Ce résultat est également atteint par un choix approprié des matériaux notamment de la lentille souple. L'invention se rapporte également à une lentille souple comprenant matériau polymère dans lequel est inclus un matériau ayant un indice de réfraction susceptible de varier sous l'action d'un signal électrique et/ou magnétique et/ou électromagnétique susceptible d'être produit par transformation de l'énergie mécanique produite lors du mouvement d'un oeil.

Toutes les caractéristiques précédemment décrites concernant la lentille dans le dispositif pour la correction visuelle s'appliquent également à une telle lentille.

On décrit maintenant un mode de réalisation de l'invention en référence aux figures annexées dans lesquelles :

- FIG. 1 représente un dispositif pour la correction visuelle au niveau d'un oeil selon un mode de réalisation de l'invention ;
- FIG. 2 représente une orientation planaire des cristaux liquides dans la lentille selon l'invention ;
- FIG. 3 représente une orientation homéotrope des cristaux liquides dans la lentille selon l'invention ;
- FIG. 4 est une courbe représentant la variation de l'orientation des cristaux liquides en fonction de la tension électrique appliquée dans la lentille selon l'invention ;
- FIG. 5 représente quatre situations des yeux d'un utilisateur pouvant activer ou non la génération d'un signal électrique suite à la détection d'un mouvement des yeux.

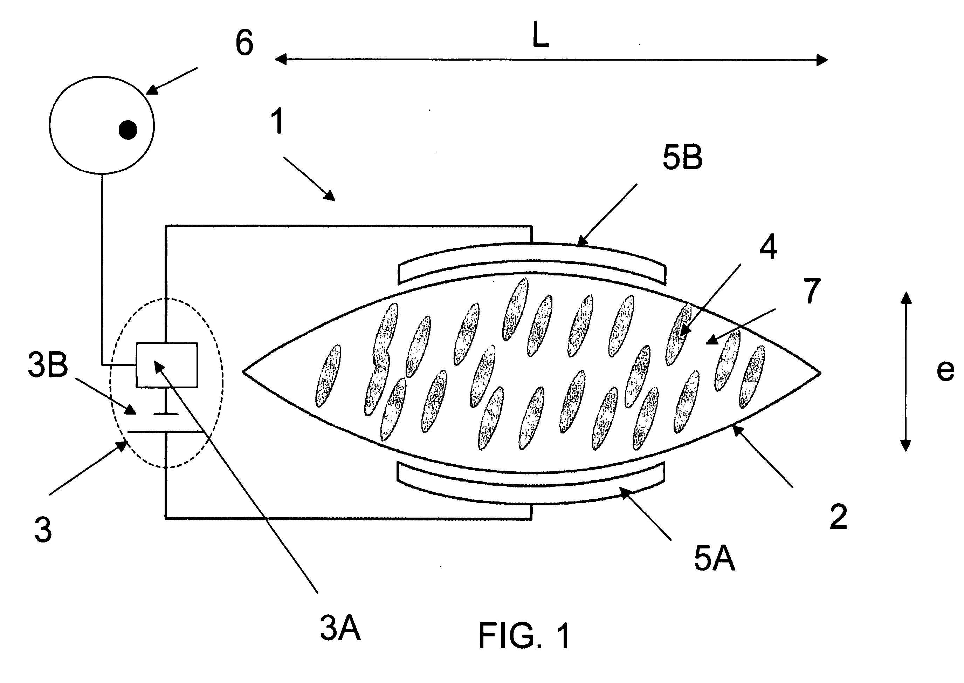

Sur la FIG. 1, un dispositif 1 pour la correction visuelle au niveau d'un oeil 6 comprend une lentille 2. La lentille 2 comprend des cristaux liquides 4 dans un polymère 7. La lentille 2 est entourée de part et d'autre par des électrodes transparentes 5A et 5B. Les tissus musculaires de l'oeil 6 sont reliés à un convertisseur 3 comprenant des moyens 3A pour détecter un mouvement de l'oeil 6, et un moyen 3B pour générer un signal électrique en réponse à ce mouvement de l'oeil. Les moyens 3A comprennent par exemple des capteurs de pression.

Le convertisseur 3 comprenant les moyens 3A pour détecter un mouvement de l'oeil 6 et générer un signal électrique en réponse à ce mouvement peut être tel que décrit dans la demande internationale WO 2004/004605. En particulier, comme décrit dans ce document, le convertisseur 3 peut comprendre une ou plusieurs jauges de contraintes, pouvant être réalisées à partir de capteurs de pression 3A absolus miniatures qui sont insérés, sous le tendon d'insertion des muscles droits externes. Il peut s'agir de microstructures millimétriques sur silicium qui sont alimentées sans contact et sans batterie, comme par induction. De tels systèmes comprennent un élément sensible, un convertisseur et un coupleur associé à une antenne secondaire permettant la télé alimentation du système et la télétransmission de la mesure de pression.

Plus précisément, l'élément sensible est une microstructure mécanique se déformant sous l'effet d'une force, en l'occurrence de la pression qu'elle subit, déformation qui provoque la modification de capacités intégrées dans l'ensemble sensible. La valeur électrique des variations de capacités est transformée en signal numérique par le convertisseur. L'élément sensible peut également enregistrer des variations de forme ou des déformations axiales telles que des extensions ou des compressions.

Grâce au convertisseur 3 tel que précédemment décrit, il est donc possible d'obtenir, en réponse à un mouvement d'un oeil, un signal électrique correspondant à une tension entre les électrodes transparentes 5A et 5B, d'environ 1 à 5 volts. La tension électrique générée est fonction du mouvement de l'oeil, ce mouvement pouvant être un mouvement d'un oeil ou un mouvement combiné de deux yeux. Dans l'exemple décrit, le convertisseur 3 est un transducteur qui transforme l'énergie mécanique due au mouvement de l'oeil en un signal électrique. D'autres modes de réalisation de ce convertisseur peuvent être envisagés tant qu'un signal électrique et/ou magnétique et/ou électromagnétique est généré en réponse à un mouvement de l'oeil.

On décrit maintenant plus en détail la lentille 2 et les cristaux liquides 4 associés dans le cadre d'une accommodation correspondant à une convergence des yeux.

La lentille 2 est une lentille biconvexe symétrique, de convergence C voisine de 21 dioptries pour 1 oeil emmétrope, d'indice moyen de réfraction n égal à 1,5, et de rayon de courbure R. Pour une lentille 2 biconvexe, on sait que la convergence est donnée par la relation $C=2(n-1)/R$, de sorte que la courbure est dans ce cas de 5 centimètres. Le diamètre de la lentille 2 est L=6 millimètres, et le rayon de courbure est 50 millimètres. On en déduit une épaisseur de la lentille de l'ordre de 180 micromètres.

Dans ce cas, si l'on désire que la convergence puisse varier d'environ 3 dioptries lors de l'ajustement de la lentille, il vient $\Delta C=(2.\Delta n)/R=3$ dioptries, soit $\Delta n=0,0075$.

Selon cet exemple, les cristaux liquides doivent donc être capables de modifier l'indice de réfraction d'une valeur égale à $\Delta n=0,0075$, ce qui est possible puisque, si ne et $n_o$ sont respectivement les indices extraordinaires et ordinaires des cristaux liquides, on a :

$$0,05 < n_e - n_o - \Delta n < 0,25$$

Selon l'invention, les cristaux liquides ont des indices ordinaires et extraordinaires respectivement égaux à 1,5 et 1,65.

Selon l'orientation des molécules de cristaux liquides dans la lentille, il est connu que l'indice apparent du milieu comprenant les cristaux liquides varie.

Pour une orientation planaire dans laquelle les molécules sont parallèles aux substrats inférieurs et supérieurs constituant la surface interne de la lentille, pour une lumière naturelle et non polarisée, il est connu que l'indice apparent est :

$$n_p = [(n_o{}^2 + n_e{}^2)/3]^{1/2} = 1,5767$$

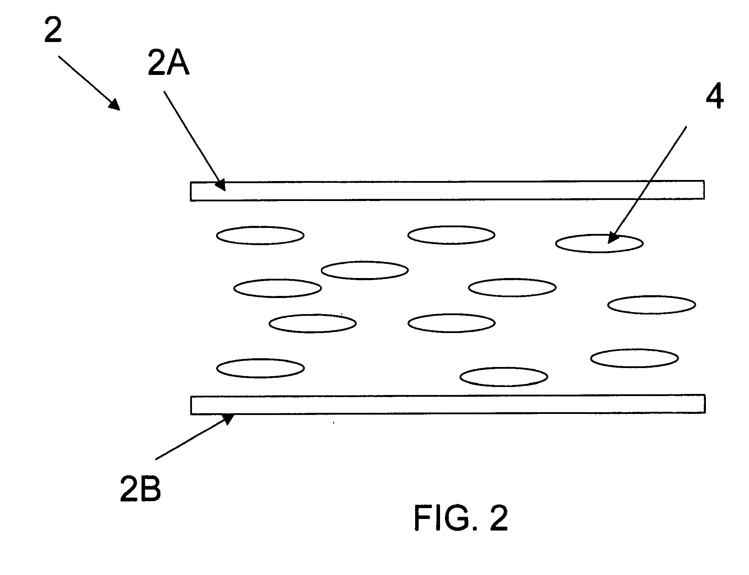

Cette orientation planaire est représentée schématiquement FIG. 2. Les cristaux liquides 4 sont placés parallèlement aux surfaces 2A et 2B de la lentille 2.

Pour une orientation homéotrope dans laquelle les molécules sont perpendiculaires aux substrats inférieurs et supérieurs constituant la surface interne de la lentille, pour une lumière naturelle et non polarisée, il est connu que l'indice apparent est :

$$n_p = n_o = 1,65$$

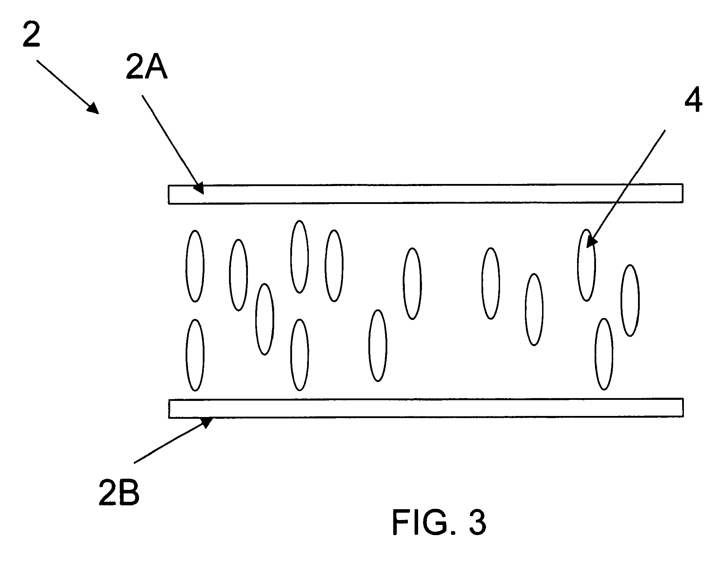

Cette orientation homéotrope est représentée schématiquement FIG. 3. Les cristaux liquides 4 sont placés perpendiculairement aux surfaces 2A et 2B de la lentille 2.

Lors d'un changement d'orientation des cristaux liquides sous l'effet d'une tension lors d'un mouvement de l'oeil, les cristaux liquides peuvent passer d'une orientation homéotrope à une orientation planaire.

Dans ce cas, la variation d'indice maximale est $n_p$-$n_o$=0,0767, ce qui est donc tout à fait acceptable pour réaliser un changement de convergence de 3 dioptries, comme précédemment mentionné. Plus précisément, selon un mode de réalisation de l'invention, on désire que le dispositif selon l'invention permette d'accroître la convergence, à rayon de courbure constant, en passant d'une vision de loin à une vision de près. L'agencement des cristaux liquides dans la lentille est alors adapté pour atteindre ce résultat.

Pour ce faire, il est nécessaire que la lentille 2 soit de faible indice pour une vision de loin, donc que les molécules de cristaux liquides soient à orientation homéotrope et de fort indice pour une vision de près, donc que les molécules de cristaux liquides soient à orientation planaire, puisque, comme on l'a vu plus haut, np est supérieur à $n_o$.

Par ailleurs, on définit l'anisotropie diélectrique des molécules de cristaux liquides pour deux positions orthogonales d'un champ électrique comme $\Delta\varepsilon$, avec :

- $\Delta\varepsilon$>0 si le moment dipolaire est dirigé selon la direction principale d'allongement des molécules ;
- $\Delta\varepsilon$<0 si le moment dipolaire est dirigé perpendiculairement à la direction principale d'allongement des molécules.

Pour avoir une variation d'indice positive par basculement des molécules vers une orientation planaire sous l'effet d'un champ électrique, on choisit donc un cristal liquide à anisotropie diélectrique négative et à orientation homéotrope en l'absence de champ. Le convertisseur 3 est alors agencé de sorte que l'oeil soit au repos en vision de loin et qu'aucun champ électrique ne soit fourni aux cristaux liquides en vision de loin.

Pour avoir une variation d'indice positive par basculement des molécules vers une orientation homéotrope sous l'effet d'un champ électrique, on choisit donc un cristal liquide à anisotropie diélectrique positive et à orientation planaire en l'absence de champ.

Pour garantir que l'orientation des molécules de cristaux liquides est bien homéotrope dans toute l'épaisseur du cristal liquide, on traite les surfaces limitant la lentille de façon connue en soi, par exemple dans le domaine des afficheurs à cristaux liquides.

On peut aussi obtenir une pré-orientation des cristaux liquides par l'application d'un champ électrique de pré-orientation lors de la fabrication de la lentille, et notamment lors de la polymérisation de la lentille.

Les mêmes procédures de traitement de surface et application d'un champ électrique de pré-orientation sont applicables pour garantir une orientation planaire des molécules dans toute l'épaisseur de la lentille

L'effet de la tension appliquée aux cristaux liquides est alors d'orienter le moment dipolaire des cristaux liquides perpendiculairement aux surfaces limitant le cristal liquide. Pour obtenir une homogénéité d'orientation en présence du champ électrique, on impose une pré-orientation aux molécules en l'absence de champ. Les cristaux liquides sont donc inclinés légèrement par rapport à la position homéotrope.

Les cristaux liquides utilisés peuvent être des cristaux liquides nématiques, soit des cristaux liquides ferroélectriques. Selon le type de cristal liquide choisi, les conditions d'orientation peuvent varier, notamment en fonction de l'anisotropie diélectrique $\Delta\varepsilon$.

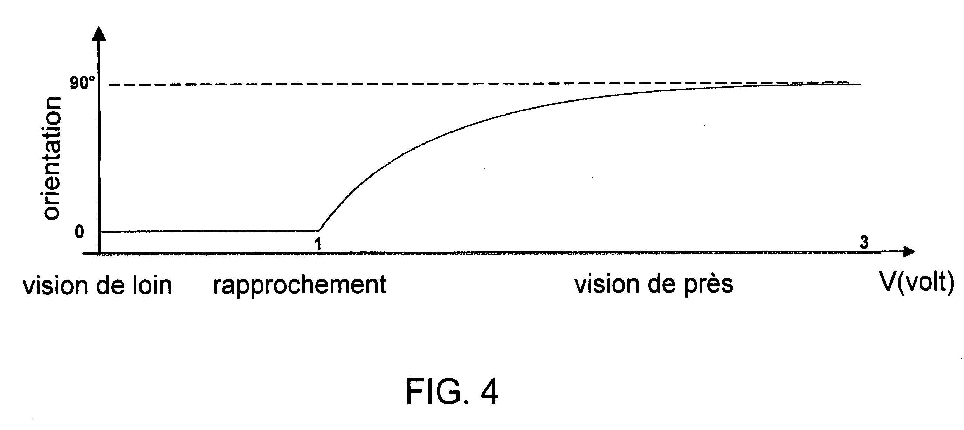

On décrit maintenant la courbe de réponse des cristaux liquides en fonction de la tension appliquée dans la lentille en référence à la FIG. 4. Cette courbe est connue en soi dans le cadre de l'effet Fréedericks. La tension seuil

$V_0$ est donnée par l'expression suivante pour un film mince correspondant à l'épaisseur de la lentille:

$$V_0 = \pi(K/\Delta\varepsilon), \text{ K étant la constante d'élasticité du cristal liquide.}$$

Au-delà de cette tension seuil, l'angle $\alpha$ donnant l'orientation d'alignement des molécules de cristaux liquides avec la surface de la lentille 2 est proportionnel au champ électrique appliqué, toujours en condition de film mince.

L'épaisseur de la lentille étant environ de 180 micromètres, la condition de film mince est satisfaite.

Pour un cristal liquide classique, la formule précédente donne une tension seuil d'environ 1 volt, et une tension maximale pour obtenir une orientation planaire de l'ordre de 3 volts.

Ces tensions sont compatibles avec l'énergie électrique qui peut être générée par le convertisseur 3 suite au mouvement de l'oeil ou des yeux.

De la sorte, lors du rapprochement entre la vision de loin et la vision de près, l'indice de la lentille n'est pas modifié. Cette caractéristique correspond à la situation pour un cristallin sain qui ne réalise pas d'accommodation avant que le point de convergence des yeux atteigne une distance d'environ 5 mètres. Ce point de début de l'accommodation est appelé punctum remotum.

Le convertisseur est alors agencé pour que, lorsque les yeux atteignent une telle convergence de 5 mètres, le signal électrique généré par le convertisseur corresponde à une tension égale à la tension seuil $V_0$. La modification de l'orientation est ensuite linéaire lorsque les yeux convergent encore plus.

On décrit maintenant plus en détail un exemple de réalisation de la lentille 2 associée aux cristaux liquides 4.

De façon générale, la lentille 2 comprend un composite d'un matériau polymère 7 et des cristaux liquides 4. Grâce à ce composite, la lentille peut être manipulée facilement sans modifier de façon irréversible l'arrangement moléculaire des cristaux liquides, ce qui rendrait le dispositif inutilisable.

Cette lentille 2 est par exemple une matrice polymère 7 de forme biconvexe gonflée par du cristal liquide 4. Une enveloppe de protection également en polymère protège éventuellement cette matrice polymère. Le polymère utilisé est un polyacrylate ayant un indice optique $n_{poly}$ sensiblement égal à l'indice optique moyen des cristaux liquides $n_{CL}$ de sorte à éviter les phénomènes de diffusion au sein de la lentille.

Selon une première variante, la lentille 2 peut également comprendre un gel de polymère 7 et de cristal liquide 4. Les cristaux liquides 4 seront alors figés dans le réseau de polymère 7 tout en restant mobiles assez facilement sous l'action du champ électrique.

Selon une deuxième variante, la lentille 2 peut également comprendre des gouttelettes de cristaux liquides 4 dans une matrice de polymère 7. Dans ce cas, les cristaux liquides 4 sont de préférence de type ferroélectriques. L'insertion de gouttelettes de polymères dans une matrice polymère est connue en soi. Le procédé de fabrication d'une telle structure est par exemple décrit dans la publication [M. BOUSSOUALEM (thèse de doctorat de l'Université du Littoral Côte d'Opale Décembre 2005) «Contribution à l'optimisation de la qualité de régulation lumineuse de films composites : étude physicochimique aux interfaces matrice-phase complexe » ; M. BOUSSOUALEN, M. ISMAILI, J.-F. LAMONOER, J.M. BUISINE, F. ROUSSEL Polarization field-effect at Liquid Crystal droplets-polymer interface Physical Review 73 (2006)].

Dans tous ces modes de réalisation, les cristaux liquides 4 sont inclus dans un matériau polymère 7 de sorte à avoir une bonne réactivité au champ électrique, tout en étant relativement stable en l'absence de champ électrique.

Dans tous ces modes de réalisation, notamment lorsqu'elle est utilisée en tant qu'implant intra-oculaire, la lentille peut comprendre une partie optique, d'où se projettent des bras appelés haptiques, qui servent à fixer l'implant dans l'oeil d'un patient.

On décrit maintenant plus en détail les électrodes 5A et 5B. Ces électrodes 5A et 5B sont des électrodes transparentes, en un matériau d'oxyde mixte indium/ étain. Elles sont agencées pour épouser la forme des surfaces de la lentille 2 et sont donc concaves. Elles sont positionnées par rapport à la lentille 2 de sorte à appliquer une tension dans la zone de la lentille 2 comprenant les cristaux liquides.

Selon une variante, ces électrodes 5A et 5B comprennent un polymère conducteur.

On décrit maintenant le dispositif 1 selon l'invention en fonctionnement.

Suite à une opération chirurgicale, la lentille 2 est positionné au niveau de l'oeil, par exemple en tant qu'implant intra-oculaire, dans le cas d'une opération de la cataracte. Le convertisseur 3 est relié aux muscles de l'oeil pour détecter un mouvement de l'oeil et générer en réponse un signal électrique. Ce signal électrique peut être fourni à la lentille 2 sous la forme d'une tension par l'intermédiaire des électrodes 5A et 5B.

Lorsque le porteur du dispositif 1 a un mouvement de l'oeil, par exemple un mouvement de convergence des deux yeux correspondant à un passage d'une vision de loin à une vision de près, le convertisseur 3 détecte ce mouvement par l'intermédiaire de capteur 3A. Une fois ce mouvement détecté, un signal électrique est généré par les moyens de génération 3B. Ce signal électrique est fourni aux électrodes 5A et 5B, de sorte qu'une tension est générée entre les électrodes 5A et 5B.

Selon la valeur de cette tension, et en fonction du graphique de réponse tel qu'illustré FIG. 4, l'orientation des cristaux liquides 4 est modifiée. Comme décrit précédemment, ces cristaux liquides peuvent être choisis pour que l'indice apparent de la lentille 2 augmente en cas de convergence des yeux de l'utilisateur.

Une fois l'indice augmenté, l'utilisateur a une vision correcte de près, sans nécessiter de lunettes supplémentaires.

On décrit maintenant des variantes de l'invention.

On a décrit le fait que le signal électrique est fourni à la lentille sous la forme d'une tension de sorte à modifier l'orientation des cristaux liquides. Il est toutefois également possible que les cristaux liquides s'orientent sous l'effet d'un champ magnétique, ce qui entraînera alors comme précédemment une modification de l'indice de réfraction de la lentille. Cet effet, semblable à l'effet Fréedericks susmentionné est connu. Pour générer le champ magnétique, on peut utiliser des transducteurs électromagnétiques au niveau des muscles de l'oeil et appliquer un champ magnétique généré par le mouvement de l'oeil à la lentille. Ce mode de réalisation possède l'avantage de ne pas nécessiter d'électrodes au niveau de la lentille.

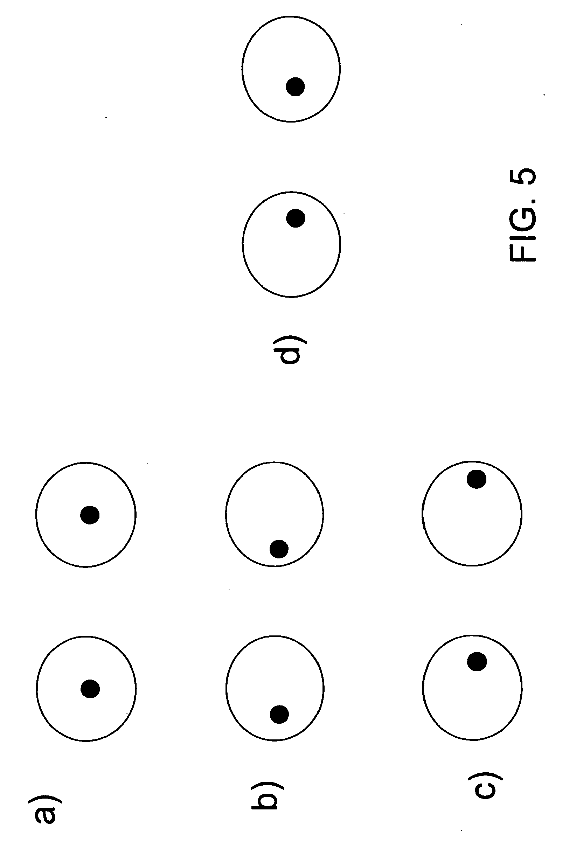

Par ailleurs, on a décrit le dispositif dans le cadre d'un mouvement particulier des yeux correspondant à un passage d'une vision de loin à une vision de près. Il est toutefois entendu que le convertisseur peut détecter tout mouvement d'un oeil ou des deux yeux, et générer le signal électrique en fonction de ce mouvement. En référence à la FIG. 5, le convertisseur pourra être agencé pour que le signal électrique soit par exemple être généré dans le cas « d » pour les deux yeux, dans les cas « b et c » pour l'oeil en convergence seule, et pour aucun des deux yeux dans le cas « a ».

## Revendications

1. Dispositif (1) pour la correction visuelle au niveau d'un oeil (6) comprenant :

   - un convertisseur (3, 3A, 3B, 5A, 5B) apte à générer un signal électrique et/ou magnétique et/ou électromagnétique en réponse à l'énergie mécanique générée par un mouvement de l'oeil;
   - une lentille souple (2) agencée pour être positionnée au niveau de l'oeil,

   le convertisseur étant agencé par rapport à la lentille de sorte que les propriétés optiques de la lentille changent sous l'action du signal électrique et/ou magnétique et/ou électromagnétique lors du mouvement de l'oeil, **caractérisé en ce que** la lentille (2) comprend un matériau polymère (7) dans lequel est inclus un matériau (4) ayant un indice de réfraction susceptible de varier sous l'action du signal électrique et/ou magnétique et/ou électromagnétique lors du mouvement de l'oeil et l'énergie pour faire varier l'indice de réfraction est générée uniquement par le mouvement de l'oeil.

2. Dispositif selon la revendication 1 dans lequel le matériau comprend des cristaux liquides (4) ayant une orientation susceptible de varier sous l'action de la tension du signal électrique et/ou magnétique et/ou électromagnétique lors du mouvement de l'oeil.

3. Dispositif selon la revendication 1 ou 2 dans lequel la lentille (2) comprend un composite du matériau polymère (7) et des cristaux liquides (4).

4. Dispositif selon la revendication précédente dans lequel le composite est une matrice polymère (7) gonflée par les cristaux liquides (4).

5. Dispositif selon la revendication 3, dans lequel le composite est un gel de polymère (7) et de cristaux liquides (4).

6. Dispositif selon la revendication 3 dans lequel le composite est une matrice de polymère (7) comprenant une dispersion de gouttelettes de cristaux liquides (4).

7. Dispositif selon l'une des revendications 2 à 6 dans lequel le polymère est un polyacrylate ayant un indice optique sensiblement égal à l'indice optique moyen des cristaux liquides.

8. Dispositif selon l'une des revendications 2 à 7 dans lequel les cristaux liquides sont des cristaux liquides nématiques.

9. Dispositif selon l'une des revendications 2 à 8 dans lequel les cristaux liquides sont des cristaux liquides ferroélectriques.

10. Dispositif selon l'une des revendications 2 à 9, dans lequel les cristaux liquides ont une anisotropie diélectrique négative et une orientation homéotrope en l'absence de champ électrique.

11. Dispositif selon l'une des revendications 2 à 10 dans lequel les cristaux liquides ont une anisotropie diélectrique positive et ont une orientation planaire en l'absence de champ électrique.

12. Dispositif selon l'une des revendications 2 à 11, dans lequel les cristaux liquides sont inclinés par rapport à une orientation homéotrope en l'absence de champ électromagnétique.

13. Dispositif selon l'une des revendications 2 à 12 dans lequel les cristaux liquides ont une orientation susceptible de varier sous l'action de la tension du signal électrique et/ou magnétique et/ou électromagnétique lors d'un mouvement de convergence de deux yeux.

14. Dispositif selon la revendication précédente dans lequel le convertisseur est agencé de sorte que l'effet de seuil de modification d'orientation des cristaux liquides est atteint lorsqu'un mouvement d'accommodation des yeux démarre au punctum remotum.

15. Dispositif selon la revendication précédente, dans lequel le convertisseur est agencé de sorte que l'effet seuil de modification d'orientation des cristaux liquides est atteint lorsque l'intersection des axes optiques des deux yeux se coupent à une distance d'environ cinq mètres par rapport aux yeux.

16. Dispositif selon l'une des revendications précédentes dans lequel le convertisseur comprend une paire d'électrodes (5A, 5B) transparentes dans le domaine du visible, les électrodes de la paire d'électrodes étant positionnées de part et d'autre de la lentille.

17. Dispositif selon la revendication 16 dans lequel les électrodes de la paire d'électrode comprennent un matériau d'oxyde mixte indium/ étain.

18. Dispositif selon la revendication 16 dans lequel les électrodes de la paire d'électrode comprennent un matériau polymère conducteur.

19. Dispositif selon l'une des revendications précédentes dans lequel le convertisseur comprend un capteur de pression (3A), un transducteur (3B) apte à transformer une pression due au mouvement de l'oeil en un signal électrique et/ou magnétique et/ou électromagnétique.

**Patentansprüche**

1. Vorrichtung (1) für die Sehkorrektur im Bereich eines Auges (6), die Folgendes umfasst :

   - einen Wandler (3, 3A, 3B, 5A, 5B), der geeignet ist, um ein elektrisches und/oder magnetisches und/oder elektromagnetisches Signal als Reaktion auf die mechanische Energie, die von einer Bewegung des Auges erzeugt wird, zu erzeugen;
   - eine biegsame Linse (2), die eingerichtet ist, um im Bereich des Auges positioniert zu sein,

   wobei der Wandler in Bezug auf die Linse derart eingerichtet ist, dass sich die optischen Eigenschaften der Linse unter der Einwirkung des elektrischen und/oder magnetischen und/oder elektromagnetischen Signals bei der Bewegung des Auges ändern,
   **dadurch gekennzeichnet, dass** die Linse (2) einen Polymerwerkstoff (7) umfasst, in dem ein Werkstoff (4) enthalten ist, der einen Brechungsindex aufweist, der unter der Einwirkung des elektrischen und/oder magnetischen und/oder elektromagnetischen Signals bei der Bewegung des Auges variieren kann und die Energie zum Variieren des Brechungsindex nur von der Bewegung des Auges erzeugt wird.

2. Vorrichtung nach Anspruch 1, bei der der Werkstoff Flüssigkristalle (4) umfasst, die eine Ausrichtung haben, die unter der Einwirkung der Spannung des elektrischen und/oder magnetischen und/oder elektromagnetischen Signals bei der Bewegung des Auges variieren kann.

3. Vorrichtung nach Anspruch 1 oder 2, bei der die Linse (2) eine Zusammensetzung des Polymerwerkstoffs (7) und der Flüssigkristalle (4) umfasst.

4. Vorrichtung nach dem vorhergehenden Anspruch, bei der das Verbundmaterial eine Polymermatrix (7) ist, die durch die Flüssigkristalle (4) aufgebläht ist.

5. Vorrichtung nach Anspruch 3, bei der das Verbundmaterial ein Gel aus Polymer (7) und Flüssigkristallen (4) ist.

6. Vorrichtung nach Anspruch 3, bei der das Verbundmaterial eine Polymermatrix (7) ist, die eine Dispersion von Tröpfchen aus Flüssigkristallen (4) umfasst.

7. Vorrichtung nach einem der Ansprüche 2 bis 6, wobei das Polymer ein Polyacrylat ist, das einen optischen Index hat, der im Wesentlichen gleich dem mittleren optischen Index der Flüssigkristalle ist.

8. Vorrichtung nach einem der Ansprüche 2 bis 7, wobei die Flüssigkristalle nematische Flüssigkristalle sind.

9. Vorrichtung nach einem der Ansprüche 2 bis 8, wobei die Flüssigkristalle ferroelektrische Flüssigkristalle sind.

10. Vorrichtung nach einem der Ansprüche 2 bis 9, wobei die Flüssigkristalle eine negative dielektrische Anisotropie und bei Fehlen eines elektrischen Felds eine homeotrope Ausrichtung haben.

11. Vorrichtung nach einem der Ansprüche 2 bis 10, bei der die Flüssigkristalle eine positive dielektrische Anisotropie und bei Fehlen eines elektrischen Felds eine planare Ausrichtung haben.

12. Vorrichtung nach einem der Ansprüche 2 bis 11, bei der die Flüssigkristalle in Bezug auf eine homeotrope Ausrichtung bei Fehlen eines elektromagnetischen Felds geneigt sind.

13. Vorrichtung nach einem der Ansprüche 2 bis 12, bei der die Flüssigkristalle eine Ausrichtung haben, die unter der Einwirkung der Spannung des elektrischen und/oder magnetischen und/oder elektromagnetischen Signals bei einer Konvergenzbewegung von zwei Augen variieren kann.

14. Vorrichtung nach dem vorhergehenden Anspruch, wobei der Wandler derart eingerichtet ist, dass die Wirkung der Ausrichtungsänderungsschwelle der Flüssigkristalle erreicht ist, wenn eine Anpassungsbewegung der Augen am Fernpunkt startet.

15. Vorrichtung nach dem vorhergehenden Anspruch, wobei der Wandler derart eingerichtet ist, dass die Wirkung der Ausrichtungsänderungsschwelle der Flüssigkristalle erreicht ist, wenn sich optische Achsen der zwei Augen in einer Entfernung von etwa 5 m in Bezug auf die Augen schneiden.

16. Vorrichtung nach einem der vorhergehenden Ansprüche, wobei der Wandler ein Paar Elektroden (5A, 5B) umfasst, die im sichtbaren Bereich durchsichtig sind, wobei die Elektroden des Elektrodenpaars zu jeder Seite der Linse positioniert sind.

17. Vorrichtung nach Anspruch 16, bei der die Elektroden des Elektrodenpaars einen gemischten Indium-/Zinn-Oxidwerkstoff umfassen.

18. Vorrichtung nach Anspruch 16, bei der die Elektroden des Elektrodenpaars einen leitenden Polymerwerkstoff umfassen.

19. Vorrichtung nach einem der vorhergehenden Ansprüche, wobei der Wandler einen Druckfühler (3A), einen Transducer (3B), der geeignet ist, um einen Druck aufgrund der Bewegung des Auges in ein elektrisches und/oder magnetisches und/oder elektromagnetisches Signal umzuwandeln, umfasst.

**Claims**

1. A device (1) for the vision correction of an eye (6) including:

   - a converter (3, 3A, 3B, 5A, 5B) which can generate an electrical and/or magnetic and/or electromagnetic signal in response to the mechanical energy generated by a movement by the eye;
   - a soft lens (2) intended to be aligned with the eye,

   the converter being positioned in relation to the lens such that the electrical and/or the magnetic and/or the electromagnetic signal generated during the movement of the eye cause the optical properties of the lens to change, **characterised in that** the lens (2) includes a polymer material (7) containing a material (4) having a refractive index that can vary under the action of the electrical and/or magnetic and/or electromagnetic signal generated during the movement of the eye and the energy for varying the refractive index is generated only by the movement of the eye.

2. A device according to claim 1, wherein the material includes liquid crystals (4) having an orientation liable to vary under the action of the voltage of the electrical and/or magnetic and/or electromagnetic signal during the movement of the eye.

3. A device according to claim 1 or 2, wherein the lens (2) includes a composite of the polymer material (7) and liquid crystals (4).

4. A device according to the preceding claim, wherein a composite is a polymer matrix (7) swollen with liquid crystals (4).

5. A device according to claim 3, wherein the composite is a polymer (7) and liquid crystals (4) gel.

6. A device according to claim 3, wherein the composite is a polymer matrix (7) including a dispersion of liquid crystal droplets (4).

7. A device according to claims 2 to 6, wherein the polymer is a polyacrylate having an optical index substantially equal to the average optical index of the liquid crystals.

8. A device according to claims 2 to 7, wherein the liquid crystal are nematic liquid crystals.

9. A device according to one of claims 2 to 8, wherein the liquid crystals are ferroelectric liquid crystals.

10. A device according to one of claims 2 to 9, wherein the liquid crystals have a negative dielectric anisotropy and a homeotropic orientation in the absence of an electrical field.

11. A device according to one of claims 2 to 10, wherein the liquid crystals have a positive dielectric anisotropy and have a planar orientation in the absence of an electrical field.

12. A device according to one of claims 2 to 11, wherein the liquid crystals are inclined in relation to an homeotrope orientation in the absence of an electromagnetic field.

13. A device according to one of claims 2 to 12, wherein the liquid crystals have an orientation liable to vary under the action of the voltage of the electrical and/or magnetic and/or electromagnetic signal during the convergence movement of both eyes.

14. A device according to the preceding claim, wherein the converter is arranged such that the threshold effect of the modification of the orientation of the liquid crystals is reached when an accommodation movement of the eyes starts at the punctum remotum.

15. A device according to the preceding claim, wherein the converter is arranged such that the threshold effect of the modification of the orientation of the liquid crystals is reached when the optical axes of both eyes intersect at a distance of approximately five metres in relation to the eyes.

16. A device according to one of the preceding claims wherein the converter includes a pair of electrodes (5A, 5B) which are transparent in the visible region, with the electrodes of the pair of electrodes being positioned on either side of the lens.

17. A device according to claim 16, wherein the electrodes of the pair of electrodes include a mixed indium/tin oxide material.

18. A device according to claim 16, wherein the electrodes of the pair of electrodes include a conducting polymer material.

19. A device according to one the preceding claims wherein the converter includes a pressure sensor (3A), a transducer (3B) able to transform a pressure resulting from the movement of the eye into an electrical and/or magnetic and/or electromagnetic signal.

FIG. 1

2

2A

4

2B

FIG. 2

FIG. 3

EP 2 120 792 B1

FIG. 4

FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004004605 A
- WO 03007851 A
- US 4373218 A
- EP 1068555 A

**Littérature non-brevet citée dans la description**

- **M. BOUSSOUALEM.** *Contribution à l'optimisation de la qualité de régulation lumineuse de films composites : étude physicochimique aux interfaces matrice-phase complexe,* Décembre 2005
- **M. BOUSSOUALEN ; M. ISMAILI ; J.-F. LAMONOER ; J.M. BUISINE ; F. ROUSSEL.** Polarization field-effect at Liquid Crystal droplets-polymer interface. *Physical Review,* 2006, 73